# EUROPEAN PATENT SPECIFICATION

(11) **EP 0 665 744 B1**
(45) Date of publication and mention of the grant of the patent: **29.12.1997**
(21) Application number: 93924327.5
(22) Date of filing: 21.10.1993
(51) Int. Cl.: A61K 9/24

(54) **STABLE EXTENDED RELEASE ORAL DOSAGE COMPOSITION**
STABILE ZUSAMMENSETZUNG FÜR EINE ORALE DOSIS MIT VERLÄNGERTER FREISETZUNG
COMPOSITION STABLE DE DOSE ADMINISTREE ORALEMENT ET A LIBERATION PROLONGEE

(30) Priority: 23.10.1992 US 965470
(43) Date of publication of application: 09.08.1995
(73) Proprietor: SCHERING CORPORATION, Kenilworth New Jersey 07033 (US)
(72) Inventor: KWAN, Henry, K., Summit, NJ 07901 (US); LIEBOWITZ, Stephen, M., Neshanic Station, NJ 08853 (US)
(74) Representative: Ritter, Stephen David
(86) International application number: PCT/US93/09873
(87) International publication number: WO 94/09761

(56) References cited:
- EP-A- 0 309 157
- EP-A- 0 311 067
- EP-A- 0 396 404
- US-A- 4 601 894

## Description

### BACKGROUND OF THE INVENTION

This invention relates to a film-coated extended release oral dosage composition containing the nasal decongestant pseudephedrine in a unique polymer matrix core and a film-coating on such core containing the non-sedating antihistamine, loratadine. The oral dosage composition of this invention is useful for treating patients showing the signs and symptoms associated with the common cold, upper respiratory diseases, allergic rhinitis and nasal congestion.

Loratadine is disclosed in USP 4,282, 233 as a non-sedating antihistamine and it is useful as an anti-allergy agent in, for example, the treatment of seasonal allergic rhinitis symptoms such as sneezing and itching.

Pseudephedrine as well as pharmaceutically acceptable acid additional salts, e.g., those of HCl or H₂SO₄, is a sympathomimetic drug recognized by those skilled in the art as a safe therapeutic agent effective for treating nasal congestion and is commonly administered orally and concomitantly with an antihistamine for treatment of nasal congestion associated with allergic rhinitis. For example, 5 mg of loratadine and l20 mg of pseudoephedrine sulfate ("PES") in a matrix core repetab tablet product is available wherein the PES is equally distributed between the repetab tablet coating and barrier-coated core and wherein all the loratadine is in the coating. When the repetab tablet is placed in a stirred 0.1N HCl solution such as found in the stomach, all of the PES, as well as all of the loratadine, present in the repetab tablet coating dissolve within a one hour period in the stirred acidic medium. None of the PES in the core dissolves in the acidic medium in that the barrier coating on the core is acid-resistant. A basic medium (a simulated inestinal fluid) is required to dissolve the core-coating and release the remaining 50% of PES over a five-hour period. The repetab product is recommended for twice-a-day dosing for effectiveness. U. S. Patent 4,990,535 and 5,100,675 disclose a twice-a-day sustained release coated tablet wherein the tablet coating comprises loratadine, a hydrophilic polymer and polyethylene glycol, and the tablet core comprises acetaminophen, pseudoephedrine or a salt thereof, a swellable hydrophilic polymer and pharmaceutically acceptable excipients. Neither the twice-a-day sustained release tablet nor the twice-a-day repetab tablet makes obvious or discloses the once-a-day oral dosage composition of this invention.

The successful development of a formulation of a loratadine-pseudoephedrine once-a-day product would be desirable, but would require achieving a release rate profile for the pseudoephedrine component over an extended period in excess of twelve hours and preferably at least 16 hours while maintaining the safety and effectiveness of loratadine. Products containing non-sedating antihistamines in combination with pseudoephedrine such as Seldane-D, a press-coated product of terfenadine and pseudoephedrine and Hismanal-D, a combination of pseudoephedrine prills and a separate astemizole tablet are known, but do not make obvious the extended release composition of this invention. Furthermore, the administration of terfenadine and astemizole products to humans has been found to cause adverse effects including cardiac arrhythmias and occurrence of these arrhythmias have increased when the terfenadine or astemizole products are co-administered with other drugs such as ketoconazole and erythromycin or upon overdose of the non-sedating anti-histamine.

It would be desirable for increased patient compliance to have an extended release oral dosage loratadine-pseudoephedrine composition effective and sale when used on a once-a-day basis for the treatment, management and/or mitigation of the signs and symptoms associated with the common cold, upper respiratory diseases, allergic rhinitis and nasal congestion.

### SUMMARY OF THE INVENTION

We have discovered that a film-coating of loratadine on a core tablet containing a pseudoephedrine salt, preferably pseudoephedrine sulfate, in a specific polymer matrix provides immediate release of loratadine and extended release of pseudoephedrine sulfate from the matrix core over a period in excess of twelve hours.

Thus the present invention provides a film-coated extended release oral dosage composition comprising:
a. a matrix core comprising:

| | mg/core |
|---|---|
| Pseudoephedrine Sulfate | 120-360 |
| Hydroxypropyl Methylcellulose 2208 l00,000 cps (100 Pa.s) | l60-480 |
| Ethylcellulose | 40-l20 |
| Dibasic Calcium Phosphate Dihydrate | 56-l64 |
| Povidone | 20-60 |
| Silicon Dioxide | 6-l2 |
| and | |
| Magnesium Stearate | 2-6 |
| Matrix Core Weight Range: | 400-l200mg |

and
b. a coating on said core comprising:

| | mg/tablet |
|---|---|
| Loratadine | 5-l5 |
| Hydroxypropyl Methylcellulose 29l0 6 cps (0.006 Pa.s) | l7-50 |
| Polyethylene Glycol 400 | 0.25-5.0 |
| Polyethylene Glycol 3350 | 3.4-l0.2 |
| Approximate Coating Weight Range: | 26-80mg |
| Approximate Composition (Matrix core and coating) Weight Range: | 426-l280mg |

In a preferred aspects, the present invention provides film-coated extended release oral dosage composition comprising a
a. a matrix core comprising:

| | mg/core |
|---|---|
| Pseudoephedrine Sulfate | 240 |
| Hydroxypropyl Methylcellulose 2208 l00,000 cps. (100 Pa.s) | l60-480 |
| Ethylcellulose | 40-l20 |
| Dibasic Calcium Phosphate Dihydrate | 56-l64 |
| Povidone | 20-60 |
| Silicon Dioxide | 6-l2 |
| and | |
| Magnesium Stearate | 2-6 |
| Approximate Matrix Core Weight Range: | 524-l082mg |

and
b. a coating on said core comprising:

| | mg/tablet |
|---|---|
| Loratadine | l0 |
| Hydroxypropyl Methylcellulose 29l0 6 cps. (0.006 Pa.s) | l7-50 |
| Polyethylene Glycol 400 | 0.25-5.0 |
| Polyethylene Glycol 3350 | 3.4-l0.l5 |
| Approximate Coating Weight Range: | 31-75mg |
| Approximate Composition(Matrix Core and Coating) Weight Range: | 555-ll57mg |

In a more preferred aspect, the present invention provides a film-coated extended release oral dosage composition comprising:
a. a matrix core comprising:

| | mg/core |
|---|---|
| Pseudoephedrine Sulfate USP | 240 |
| Hydroxypropyl Methylcellulose 2208 USP l00,000 cps (100 Pa.s) | 320 |
| Ethylcellulose NF Type 7 | 80 |
| Dibasic Calcium Phosphate USP Dihydrate | l08 |
| Povidone USP | 40 |
| Silicon Dioxide NF | 8 |
| and | |
| Magnesium Stearate NF | 4 |
| Approximate Matrix Core Weight: | 800mg |

and
b. a coating upon said core comprising:

| | mg/tablet |
|---|---|
| Loratadine, Micronized | l0 |
| Hydroxypropyl Methylcellulose 29l0 USP 6 cps (0.006 Pa.s) | 33 |
| Polyethylene Glycol 400 NF | 0.67 |
| Polyethylene Glycol 3350 NF | 6.75 |
| Color Dispersion (Solids) | 6.25 |
| Approximate Coating Weight: | 57mg |
| Approximate Composition (Matrix Core and Coating) Weight: | 857mg |

### DETAILED DESCRIPTION OF THE INVENTION

We have discovered a unique oral dosage composition containing a specific selection of ingredients including specific amounts of a pseudoephedrine salt, preferably pseudoephedrine sulfate in a polymer matrix core and of loratadine in an immediate release polymer film coating on the core. The oral dosage composition of this invention provides (l) immediate release (i.e., within one hour after oral administration to a patient) of the total dose of loratadine to maintain the once-a-day efficacy of loratadine (2) the extended release of pseudoephedrine sulfate from the matrix polymer cover over a period of at least l2 preferably l2 to l6 hours and more preferably at least l6 hours from oral administration (3) reasonable dose size for enhancing patients' compliance and (4) a shelf life of at least 24 months.

In the course of development of the oral dosage composition of this invention, it was discovered that the selection of the specific polymers and of the specific ratios of such polymers for the polymer matrix core was critical to achieve the desired extended release period of at least l2 hours, preferably l2 to l6 hours and more preferably for at least l6 hours for pseudoephedrine sulfate. For example, the use of hydroxypropyl methyl cellulose 4,000 cps or l5,000 cps (4 or 15 Pa.s) as polymers in the matrix core did not provide this more preferred extended release period of at least 16 hours for dose of pseudoephedrine sulfate. We discovered that only by selecting for inclusion into the matrix core specific weight ratios of three specific polymers was the desired pseudoephedrine release profile achieved. Only by combining (1) four parts by weight of hydroxypropyl methyl cellulose 2208 USP, l00,000 cps (100 Pa.s) with (2) one part by weight of ethyl cellulose together with (3) one-half part by weight of povidone as a secondary binder was the more preferred extended release profile of at least 16 hours for pseudoephedrine sulfate from the matrix core achieved. The matrix core also contains specific amounts of silicon dioxide as a glidant and magnesium stearate as a lubricant. The tablet hardness 22 ± 6 Strong-Cobb Units (SCU) is not greatly affected by the higher level of lubricant (6mg/tablet) but it is preferred to maintain the lubricant level at l/l0 part by weight of lubricant to one part by weight of povidone as secondary binder.

The hydroxyl propyl methyl cellulose 29l0 acts as a film-forming agent in the film coating, and the polyethylene glycols act as plasticizers. Other suitable film-forming polymers which may be used include hydroxypropyl cellulose, methyl hydroxyethyl cellulose and sodium carboxymethyl cellulose.

The oral dosage composition of this invention also provides a shelf life of more than 24 months, e.g., up to 36 and 48 months so long as the tablets are stored in standard package at between 2° and 30° C in an ambient environment.

In the preparation of the tablet core the povidone is dissolved in a mixture of alcohol and water. The pseudoephedrine sulfate, hydroxypropyl methylcellulose 2208 USP, l00,000 cps (100 Pa.s), ethylcellulose, and dibasic calcium phosphate are blended and granulated with the alcoholic water solution containing povidone. The granulation is milled, and dried to a loss on drying between 0.5 to 2.0%.

The dried granulation is milled and blended with requisite amounts of silicon dioxide and magnesium stearate. The final blend is compressed to produce the oral dosage composition in the preferred form of a tablet.

The coating is normally applied to the tablet cores in the following manner:
Cores are charged into a suitable coating pan. A water dispersion of hydroxypropyl methylcellulose 29l0 USP and polyethylene glycol 3350 NF is applied to the cores. These sub-coated cores are then coated with a dispersion of loratadine, hydroxypropyl methylcellulose 29l0 USP, polyethylene glycol 3350 NF and white color dispersion. This is followed by an application of polishing coating dispersion containing hydroxypropyl methylcellulose and polyethylene glycol 400 NF. The coated tablets are then branded (with black ink) and packaged in plastic bottles and blisters for storage at a temperature between 2° and 30°C in an ambient environment

### EXAMPLE l

This example illustrate preparation of the preferred oral dosage composition of this invention. The ingredients and specific amounts thereof are listed below.

### l. Tablet Core

### A. Method of Manufacture

l. Dissolve povidone in a mixture of alcohol and water.
2. Combine the pseudoephedrine sulfate, hydroxypropyl methylcellulose 2208, ethylcellulose and dibasic calcium phosphate, dihydrate in a suitable mixing bowl and blend.
3. Granulate the blend from Step 2 with the solution from Step. l. pass the wet granulation through a screen.
4. Dry the granulation to a loss on drying between 0.5 to 2.0% as determined by a moisture balance or equivalent.
5. Pass the dried granules through a screen.
6. Add the requisite amount of silicon dioxide and magnesium stearate to the dried, milled granules and blend.
7. Compress the blend on a suitable tablet press.

During the compression operation, representative samples of the cores are taken and in-process tests are performed.

The core matrix meets the following specification:
Weight: 800 ± 5% (mg)
Thickness: 0.280 ± 0.0l0 inches
Hardness: 22 ± 6 Strong-Cobb Units

The cores are coated in the following manners:

### A. Preparation of Coating Dispersions and Solutions

### l. Sub-Coating Solution

(l) Disperse hydroxypropyl methylcellulose USP 29l0 and polyethylene glycol 3350 in a portion of hot purified water.
(2) Add the remainder of the purified water and cool the solution to room temperature.

### 2. Active Coating Dispersion

(l) Disperse hydroxypropyl methylcellulose USP 29l0 and polyethylene glycol 3350 in a portion of hot purified water. Add additional water and cool the dispersion to room temperature.
(2) Disperse Loratadine in the remaining portion of room temperature purified water. Combine with hydroxypropyl methylcellulose/polyethylene glycol dispersion (Step l).
(3) Add white color dispersion. Mix until uniform.

### 3. Polishing Coating Solution

(l) Disperse hydroxypropyl methylcellulose USP 29l0 and polyethylene glycol 400 in a portion of hot purified water.
(2) Add the remainder of the purified water and cool the solution to room temperature.

### B. Coating of Tablet Core

(l) Charge the requisite quantity of tablet cores to a suitable coating pan.
(2) Apply the sub-coating solution.
(3) Quantitatively apply the active coating dispersion
(4) Apply the polishing coating solution

### C. Branding

(l) Brand the coated tablets with black imprinting ink.

The preferred composition of-the tablet core and coating is given below

| Tablet Matrix Core | |
|---|---|
| | mg/core |
| Pseudoephedrine Sulfate USP | 240 |
| Hydroxypropyl Methylcellulose 2208 USP l00,000 cps (100 Pa.s) | 320 |
| Ethylcellulose NF Type 7 | 80 |
| Dibasic Calcium Phosphate USP Dihydrate | l08 |
| Povidone USP | 40 |
| Silicon Dioxide NF | 8 |
| Magnesium Stearate NF | 4 |
| Approximate Matrix Core Weight: | 800mg |

| Tablet Coating | |
|---|---|
| | mg/tablet |
| Loratadine, Micronized | l0 |
| Hydroxypropyl Methylcellulose 29l0 USP 6 cps (0.006 Pa.s) | 33 |
| Polyethylene Glycol 400 NF | 0.67 |
| Polyethylene Glycol 3350 NF | 6.75 |
| Color Dispersion (Solids) | 6.25 |
| Imprinting Ink | ---- |
| Approximate Coating Weight: | 57mg |
| Approximate Tablet (Matrix Core and Coating) Weight: | 857mg |

The in vitro dissolution profile of the tablet of Example l was measured in a stirred 0.lN HCl solution at 37°C (1st hour) and thereafter (for an additional 15 hours) in a stirred phosphate buffer having a pH of 7.5 at 37°C. The loratadine in the coating was dissolved within the first hour and the total dose of pseudoephedrine sulfate in the core was slowly released via erosion and dissolution mechanisms over a period of at least l6 hours (see Table A hereinafter).

Similar results would be expected if a decongestant effective amount of another pharmaceutically acceptable pseudoephedrine salt, e.g., pseudoephedrine chloride were used in place of pseudoephedrine sulfate.

**TABLE A**

| **IN VITRO DISSOLUTION PROFILE OF LORATADINE AND PSEUDOEPHEDRINE SULFATE ("PES") FROM EXTENDED RELEASE TABLETS OF EXAMPLE l** | | |
|---|---|---|
| Time, Hour | % Dissolved | |
| | Loratadine^{a} | PES^{b} |
| l | 97 | 25 |
| 2 | -- | 37 |
| 4 | -- | 53 |
| 6 | -- | 64 |
| 8 | -- | 74 |
| 10 | -- | 82 |
| 12 | -- | 88 |
| 16 | -- | 96 |

| | | |
|---|---|---|
| a Medium: 1000 ml 0.lN HCL, 37°C; USP Paddle, l00 rpm; average of l2 tablets. | | |
| b Medium: 1000 ml purified water, 37°C; USP Paddle, l00 rpm; average of l2 tablets. | | |

## Claims

1. A film-coated extended release oral dosage composition comprising:
a) a matrix core comprising:
| | mg/core |
|---|---|
| Pseudoephedrine Sulfate | 120-360 |
| Hydroxypropyl Methylcellulose 2208 100,000 cps (100 Pa.s) | 160-480 |
| Ethylcellulose | 40-120 |
| Dibasic Calcium Phosphate Dihydrate | 56-164 |
| Povidone | 20-60 |
| Silicon Dioxide | 6-12 |
| and | |
| Magnesium Stearate | 2-6 |
| Matrix Core Weight Range: | 400-1200mg |
and
b) a coating on said core comprising: ,
| | mg/tablet |
|---|---|
| Loratadine | 5-15 |
| Hydroxypropyl Methylcellulose 2910 6 cps (0.006 Pa.s) | 17-50 |
| Polyethylene Glycol 400 | 0.25-5.0 |
| Polyethylene Glycol 3350 | 3.4-10.2 |
| Approximate Coating Weight Range: | 26-80mg |
| Approximate Composition (Matrix Core and Coating) Weight Range: | 426-1280mg |

2. The oral dosage composition of Claim 1 wherein 240 mg. of pseudoephedrine sulfate is in the matrix core and 10 mg. of loratadine is in the coating.

3. A film-coated extended release oral dosage composition according to Claim 2 comprising:
a) a matrix core comprising:
| | mg/core |
|---|---|
| Pseudoephedrine Sulfate USP | 240 |
| Hydroxypropyl Methylcellulose 2208 USP 100,000 cps (100 Pa.s) | 320 |
| Ethylcellulose NF Type 7 | 80 |
| Dibasic Calcium Phosphate USP Dihydrate | 108 |
| Povidone USP | 40 |
| Silicon Dioxide NF | 8 |
| and | |
| Magnesium Stearate NF | 4 |
| Approximate Matrix Core Weight: | 800mg |
and
b) a coating upon said core comprising:
| | mg/tablet |
|---|---|
| Loratadine, Micronized | 10 |
| Hydroxypropyl Methylcellulose 2910 USP 6 cps (0.006 Pa.s) | 33 |
| Polyethylene Glycol 400 NF | 0.67 |
| Polyethylene Glycol 3350 NF | 6.75 |
| Color Dispersion (Solids) | 6.25 |
| Approximate Coating Weight: | 57mg |
| Approximate Composition (Matrix Core and Coating) Weight: | 857mg |

## Patentansprüche

1. Filmbeschichtete orale Dosierungszusammensetzung mit verzögerter Wirkstofffreisetzung, umfassend:
a) einen Matrixkern, umfassend:
| | mg/Kern |
|---|---|
| Pseudoephedrinsulfat | 120-360 |
| Hydroxypropylmethylcellulose 2208 100 000 cP (100 Pa·s) | 160-480 |
| Ethylcellulose | 40-120 |
| zweibasiges Calciumphosphat-Dihydrat | 56-164 |
| Povidon | 20-60 |
| Siliciumdioxid | 6-12 |
| und | |
| Magnesiumstearat | 2-6 |
| Matrixkerngewichtsbereich: | 400-1200 mg |
sowie
b) eine Beschichtung auf dem Kern, umfassend:
| | mg/Tablette |
|---|---|
| Loratadin | 5-15 |
| Hydroxypropylmethylcellulose 2910 6 cP (0,006 Pa·s) | 17-50 |
| Polyethylenglycol 400 | 0,25-5,0 |
| Polyethylenglycol 3350 | 3,4-10,2 |
| ungefährer Beschichtungsgewichtsbereich: | 26-80 mg |
| ungefährer Gewichtsbereich der Zusammensetzung (Matrix und Beschichtung): | 426-1280 mg. |

2. Orale Dosierungszusammensetzung gemäß Anspruch 1, wobei im Matrixkern 240 mg Pseudoephedrinsulfat vorhanden sind und in der Beschichtung 10 mg Loratidin vorhanden sind.

3. Filmbeschichtete orale Dosierungszusammensetzung mit verzögerter Wirkstofffreisetzung gemäß Anspruch 2, umfassend:
a) einen Matrixkern, umfassend:
| | mg/Kern |
|---|---|
| Pseudoephedrinsulfat, USP | 240 |
| Hydroxypropylmethylcellulose 2208, USP, 100 000 cP (100 Pa·s) | 320 |
| Ethylcellulose NF Typ 7 | 80 |
| zweibasiges Calciumphosphat, USP, Dihydrat, | 108 |
| Povidon, USP | 40 |
| Siliciumdioxid, NF | 8 |
| und | |
| Magnesiumstearat, NF | 4 |
| ungefähres Matrixkerngewicht: | 800 mg |
sowie
b) eine Beschichtung auf dem Kern, umfassend:
| | mg/Tablette |
|---|---|
| Loratadin, mikronisiert | 10 |
| Hydroxypropylmethylcellulose 2910, USP, 6 cP (0,006 Pa·s) | 33 |
| Polyethylenglycol 400, NF | 0,67 |
| Polyethylenglycol 3350, NF | 6,75 |
| Farbdispersion (Feststoffe) | 6,25 |
| ungefähres Beschichtungsgewicht: | 57 mg |
| ungefähres Gewicht der Zusammensetzung (Matrix und Beschichtung): | 857 mg. |

## Revendications

1. Composition pour dosage oral à libération étendue revêtue d'un film comprenant:
a) un coeur de matrice comprenant:
| | mg/coeur |
|---|---|
| Pseudoéphédrine Sulfate | 120-360 |
| Hydroxypropyl Méthylcellulose 2208 100 000 cps (100 Pa.s) | 160-480 |
| Ethylcellulose | 40-120 |
| Calcium Phosphate Dihydrate Dibasique | 56-164 |
| Povidone | 20-60 |
| Dioxyde de silicium | 6-12 |
| et | |
| Magnésium Stéarate | 2-6 |
| Intervalle de poids du coeur de la matrice: | 400-1200mg |
et
b) un revêtement sur ledit coeur comprenant:
| | mg/comprimé |
|---|---|
| Loratadine | 5-15 |
| Hydroxypropyl Méthylcellulose 2910 6 cps (0,006 Pa.s) | 17-50 |
| Polyéthylène Glycol 400 | 0,25-5,0 |
| Polyéthylène Glycol 3350 | 3,4-10,2 |
| Intervalle de poids approximatif du revêtement: | 26-80mg |
| Intervalle de poids approximatif de la composition (coeur de la matrice et revêtement): | 426-1280mg |

2. Composition pour dosage oral selon la revendication 1 dans laquelle 240mg de pseudoéphédrine sulfate sont dans le coeur de la matrice et 10mg de loratadine sont dans le revêtement.

3. Composition pour dosage oral à libération étendue revêtu d'un film selon la revendication 2 2 comprenant:
a) un coeur de matrice comprenant:
| | mg/coeur |
|---|---|
| Pseudoéphédrine Sulfate USP | 240 |
| Hydroxypropyl Méthylcellulose 2208 USP 100 000 cps (100 Pa.s) | 320 |
| Ethylcellulose NF Type 7 | 80 |
| Calcium Phosphate Dihydrate Dibasique USP | 108 |
| Povidone USP | 40 |
| Dioxyde de silicium NF | 8 |
| et | |
| Magnésium Stéarate NF | 4 |
| Poids approximatif du coeur de la matrice: | 800mg |
et
b) un revêtement sur ledit coeur comprenant:
| | mg/comprimé |
|---|---|
| Loratadine, micronisée | 10 |
| Hydroxypropyl Méthylcellulose 2910 USP 6 cps (0,006 Pa.s) | 33 |
| Polyéthylène Glycol 400 NF | 0,67 |
| Polyéthylène Glycol 3350 NF | 6,75 |
| Dispersion de coloration (solides) | 6,25 |
| Poids approximatif du revêtement: | 57mg |
| Poids approximatif de la composition (coeur de matrice et revêtement): | 857mg |
